# EUROPÄISCHE PATENTSCHRIFT

(11) **EP 1 585 450 B1**
(45) Veröffentlichungstag und Bekanntmachung des Hinweises auf die Patenterteilung: **19.11.2008**
(21) Anmeldenummer: 03732417.5
(22) Anmeldetag: 19.05.2003
(51) Int. Cl.: A61B 17/86, A61B 17/80, A61B 17/70

(54) **MEDIZINISCHES IMPLANTAT MIT EINER GESICHERTEN KNOCHENSCHRAUBE**
MEDICAL IMPLANT COMPRISING A SECURED BONE SCREW
IMPLANT MEDICAL COMPRENANT UNE VIS A OS SOLIDEMENT FIXEE

(30) Priorität: 23.01.2003 US 349175
(43) Veröffentlichungstag der Anmeldung: 19.10.2005
(73) Patentinhaber: Cervitech Inc., Rockway, NJ 07866 (US)
(72) Erfinder: KELLER, Arnold, 23863 Kayhude (DE)
(74) Vertreter: Glawe, Delfs, Moll
(86) Internationale Anmeldenummer: PCT/EP2003/005256
(87) Internationale Veröffentlichungsnummer: WO 2004/064655

(56) Entgegenhaltungen:
- FR-A- 2 802 082
- US-A- 4 754 749
- US-A- 5 275 601
- US-A1- 2002 022 843

## Beschreibung

Bei medizinischen Implantaten, die am Knochen mittels einer Knochenschraube zu befestigen sind, besteht häufig die Forderung, das Zurückwandern der Schraube aus der ihr zugedachten Stellung zu verhindern, damit nicht angrenzende Organe dadurch gefährdet werden können. Es genügt nicht, die Schrauben gegen Drehung in Löserichtung zu sichern (US-A-5275601), weil die Gefahr besteht, daß sie sich bei lockerem Eingriff ihrer Gewindegänge in das Knochenmaterial ohne wesentliche Drehung aus dem Knochen und dem Implantat axial zurück bewegen. Ferner ist es bekannt (US-A-2002/0022843, FR-A-2802082), bei einer Knochenschraube für eine Knochenplatte am Kopf der Schraube und in der zugehörigen Bohrung der Knochenplatte zusammenwirkende Gewindegänge vorzusehen, die eine geringere Steigung als das Knochengewinde aufweisen. Dadurch wird die Reibung, die einer Rückdrehung der Schraube entgegenwirkt, vergrößert. Das Lösen der Schraube wird dadurch erschwert, aber nicht verhindert. Schließlich ist es bekannt, die Schrauben dadurch zu sichern, daß am Implantat Abdeckeinrichtungen vorgesehen sind, die nach dem Eindrehen der Schraube über den Schraubenkopf gesetzt werden, um dessen Rückbewegung auszuschließen (WO 97/20526, WO 01/26567, WO 99/56653). Solche Abdeckeinrichtungen nehmen aber zusätzlichen Raum ein, was mitunter unerwünscht ist.

Der Erfindung liegt die Aufgabe zugrunde, bei einer Knochenschraube mit Rückdrehsicherung das axiale Auswandern der Schraube aus dem Implantat zu verhindern.

Die erfindungsgemäße Lösung besteht in den Merkmalen des Anspruchs. Demnach ist vorgesehen, daß die Knochenschraube durch eine Bohrung des Implantats geführt ist, die mit einem zum Gewinde der Schraube passenden Gewinde ausgerüstet ist, daß der Schraubenschaft zwischen den Schraubenkopf und dem Gewinde einen gewindefreien Abschnitt aufweist, dessen Länge mindestens so groß ist wie die Länge der Gewindebohrungen und daß das Implantat und die Schraube mit Mitteln ausgestattet sind, die die Rückdrehung der Schraube hemmen. Wenn die Schraube sich in der ihr zugedachten Stellung befindet, liegt der gewindefreie Abschnitt des Schraubenschafts in der Gewindebohrung des Implantats. Die Gewinde der Bohrung und der Schraube greifen in diesem Zustand nicht ineinander. Die Sicherung der Schraube gegen Rückbewegung besteht darin, daß das Schraubengewinde nicht axial durch die Gewindebohrung hindurchtreten kann. Die einander zugewendeten letzten Gänge des Bohrungsgewindes und des Schraubengewindes bilden axiale Anschläge, die die Rückbewegung der Schraube verhindern. Ein Austreten der Schraube aus dem Implantat wäre nur dann denkbar, wenn das Schraubengewinde sich durch Drehung in das Gewinde der Bohrung hineinbewegen könnte. Dies aber wird verhindert durch die Rückdrehsperre.

Die Erfindung wird im folgenden näher unter Bezugnahme auf die Zeichnungen erläutert, die ein vorteilhaftes Ausführungsbeispiel veranschaulicht. Es zeigen:
- Fig. 1: einen Schnitt durch eine Endoprothese gemäß Linie I - I der Figur 2,
- Fig. 2: eine Ventralansicht der Prothese,
- Fig. 3: ein Schnitt gemäß Linie III-III der Figur 2 und
- Fig. 4: einen Querschnitt durch die in der Anordnung verwendete Knochenschraube mit Blick auf die schaftseitige Fläche des Schraubenkopfs.

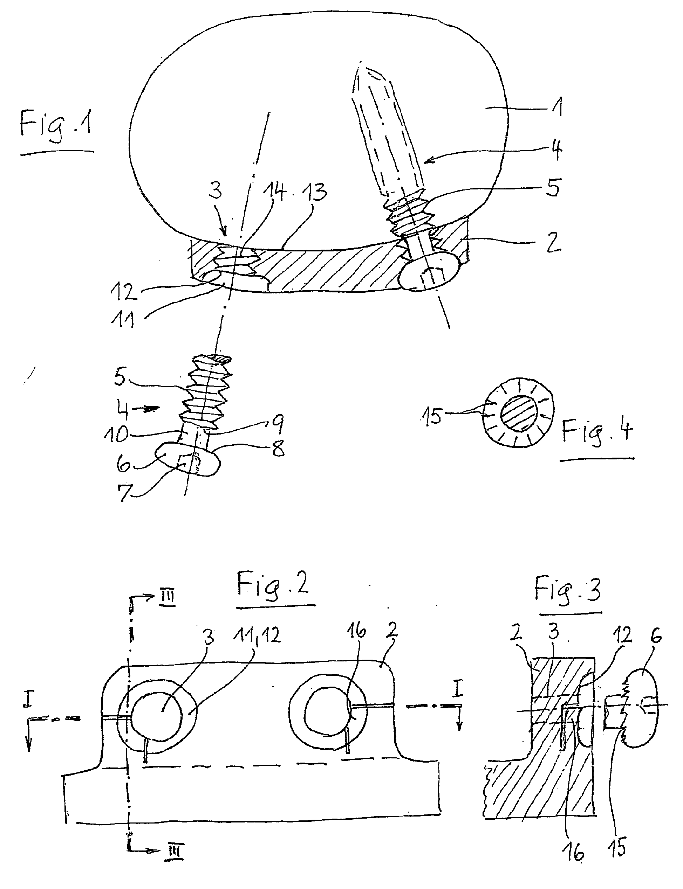

Man erkennt eine Endoprothese mit einer Platte 1, an deren Rand ein Flansch 2 angeordnet ist, der zwei Bohrungen 3 enthält, die zur Aufnahme von Knochenschrauben 4 bestimmt sind, die den Flansch 2 an der Oberfläche eines Knochens befestigen sollen. Zur Verankerung im Knochen haben die Knochenschrauben ein Gewinde 5, das in dem spitzennahen Abschnitt der rechts in Figur 1 erscheinenden Schraube lediglich durch Strichelung angedeutet ist. Am anderen Ende ist der Schaft der Schraube 4 mit einem Kopf 6 versehen, der Flächen für den Angriff eines Drehwerkzeugs aufweist, die im dargestellten Beispiel als Innensechskant 7 ausgebildet sind. Zwischen der dem Schaft zugewendeten Stirnfläche 8 des Schraubenkopfs 6 und dem benachbarten Ende 9 des Gewindes 5 befindet sich ein gewindeloser Abschnitt 10.

Die zugehörige Bohrung 3 kann (aber muß nicht) eine Einsenkung 11 zur vollständigen oder teilweisen Aufnahme des Schraubenkopfs 6 enthalten, die eine Stirnfläche 12 für die Anlage der hinteren Stirnfläche 8 des Schraubenkopfs 6 bildet. Der Bohrungsabschnitt zwischen der Stirnfläche 12 und der Rückfläche 13 des Flansch 2 der Bohrung enthält ein Gewinde 14, das zu dem Gewinde 5 der Schraube 4 paßt. Die Länge des Gewindeabschnitts in der Bohrung zwischen den Flächen 12 und 13 ist höchstens so groß wie die axiale Länge des gewindelosen Abschnitts 10 der Schraube und nicht wesentlich kürzer. Der Durchmesser des gewindelosen Abschnitts 10 der Schrauben ist nicht größer als der freie Kernquerschnitts in der Bohrung 3. Das Schraubengewinde 5 ist demnach nach dem vollständigen Einschrauben der Schraube 4 vom Gewinde 14 der Bohrung 3 frei. So kann die Schraube frei gedreht werden, wie es erforderlich ist, um den Flansch 2 durch Festdrehen der Schraube im Knochen ganz an dessen Oberfläche heranzuziehen.

Das hintere Ende 9 des Schraubengewindes 5 und das ihm zugewendete Ende des Gewindes 14 in der Bohrung 3 bilden Anschläge, die eine axiale, drehungsfreie Rückbewegung der Schraube 4 aus dem Flansch 2 des Implantats unmöglich machen. Damit auch die Rückdrehung verhindert wird, sind die Schraube und das Implantat mit einer Drehverhinderungseinrichtung versehen. Diese kann beliebiger, sehr einfacher Art sein, wie es in der Technik allgemein für die Rückdrehverhinderung von Schrauben bekannt ist. Beispielsweise kann der Schraubenkopf mit einer Sägezahnung 15 auf seiner schaftseitigen Stirnfläche versehen sein, um mit entsprechenden Organen des Flanschs 2 rückdrehverhindernd zusammenzuwirken. Im vorliegenden Beispiel sind die rückdrehverhindernden Organe in Figur 3 als eine Federzunge 16 angedeutet, die aus der Grundfläche 12 der Einsenkung 11 des Flanschs federnd hervortritt, so daß ihre Kante in Eingriff mit den Sägezähnen 15 kommen kann. Die Anordnung ist so getroffen, daß die Schraube nur in derjenigen Richtung gedreht werden kann, in welcher sie in den Knochen eingedreht wird. Wenn man versucht, sie in der Löserichtung zu drehen, kommt die Stirnkante der Lamelle 16 mit den Zähnen 15 in Eingriff und verhindert eine weitere Rückdrehung.

Das Gewinde 14 in der Bohrung 3 braucht keine wesentliche Länge zu haben. Es kann - im Gegenteil - extrem kurz sein. Es braucht nicht einmal ein vollständig umlaufender Gewindegang vorhanden zu sein. Es genügt beispielsweise die Andeutung eines Gewindegangs in Form eines Vorsprungs an einer Seite der Bohrung, der gerade groß genug ist, um den axialen Durchgang des mit Gewinde versehenen Teils der Schraube zu verhindern.

Die Rückdrehverhinderung soll wirksam sein, sobald sich die Schraube 4 mit ihrem gewindelosen Abschnitt 10 in der Bohrung 3 befindet. Der Schraubenkopf 6 muß in diesem Zustand also so nahe dem Flansch 2 sein, daß die Stirnkante der Lamelle 16 in die Zahnung 15 des Schraubenkopfs 6 eingreift. Dies wird durch den Anschlag des Gewindeendes 9 an dem letzten Gang des Bohrungsgewindes 14 gewährleistet. Mit anderen Worten muß der Abstand des Gewindeendes 9 von der Zahnung 15 geringer sein als der Abstand des hinteren Endes des Bohrungsgewindes 14 von der Stirnkante der Federzunge 16 in deren entspannten, am weitesten vorragenden Zustand. Dabei ist zu berücksichtigen, daß der Anschlag der Gewindeenden aneinander die Relativposition des Flanschs und der Schraube nur mit einer Unsicherheit einer Schraubenganghöhe bestimmt. Die Federstrecke der Stirnkante der Federzunge 16 muß also jedenfalls größer sein als eine Gewindeganghöhe. Diese Bedingung kann dadurch erleichtert werden, daß die die Rückdrehverhinderung bewirkende Rasteinrichtung (Zahnung und Federzunge) nicht an der hinteren Stirnfläche sondern an der Umfangsfläche des Schraubenkopfs oder im Übergangsbereich zwischen der hinteren Stirnfläche und der Umfangsfläche vorgesehen wird.

## Patentansprüche

1. Medizinisches Implantat mit einer durch eine Bohrung (3) des Implantats (1, 2) geführten Knochenschraube (4), für die eine Rückdrehverhinderungseinrichtung (15, 16) vorgesehen ist und die zwischen ihrem Kopf (6) und ihrem Gewinde (5) einen gewindefreien Abschnitt (10) aufweist, dessen Durchmesser nicht größer als der freie Durchmesser der Bohrung (3) ist, **dadurch gekennzeichnet, daß** die Bohrung (3) ein zu dem Gewinde (5) der Schraube (4) passendes Gewinde (14) enthält und die Länge des gewindefreien Abschnitts (10) mindestens so groß ist wie die Länge der das Gewinde (14) enthaltenden Bohrung (3).

## Claims

1. Medical implant with a bone screw (4) which is guided through a bore (3) of the implant (1, 2), for which screw an arrangement (15, 16) preventing backward turning is provided and which screw, between its head (6) and its thread (5), has an unthreaded portion (10) whose diameter is not greater than the free diameter of the bore (3), **characterized in that** the bore (3) has a thread (14) matching the thread (5) of the screw (4), and the length of the unthreaded portion (10) is at least as long as the length of the bore (3) having the thread (14).

## Revendications

1. Implant médical comprenant une vis à os (4) guidée à travers un perçage (3) de l'implant (1, 2), pour laquelle est prévu un dispositif anti-dévissage (15, 16), et qui présente, entre sa tête (6) et son filetage (5), une portion non filetée (10), dont le diamètre n'est pas supérieur au diamètre libre du perçage (3), **caractérisé en ce que** le perçage (3) contient un filetage (14) adapté au filetage (5) de la vis (4) et la longueur de la portion sans filetage (10) est au moins aussi grande que la longueur du perçage (3) contenant le filetage (14).
